# EUROPEAN PATENT APPLICATION

(11) **EP 1 655 284 A1**
(43) Date of publication of application: **10.05.2006**
(21) Application number: 04105304.2
(22) Date of filing: 26.10.2004
(51) Int. Cl.: C07D 207/48, C07D 401/12, A61K 31/40, A61P 35/04

(54) **2-Phenylsulfopyrroles**

(71) Applicant: Aponetics AG, 4108 Witterswil (CH)
(72) Inventor: Bachmann, Felix, 4052, Basel (CH); Strebel, Alessandro, 4104, Oberwil (CH); Eberle, Martin, 4103, Bottmingen (CH); Obrecht, Daniel, 4112, Bättwil (CH); Zhang, Kesheng, 4123, Allschwil (CH)
(74) Representative: Becker, Konrad

(57) **Abstract**

The invention relates to novel 2-phenylsulfopyrroles of formula (I) 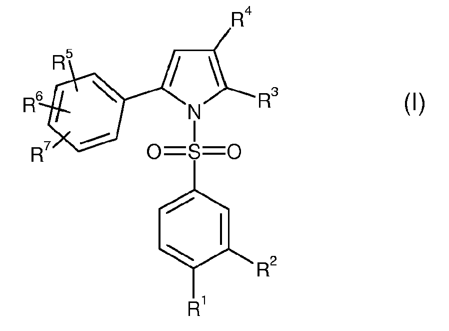 wherein R¹ represents lower alkyl, halo-lower alkyl, cycloalkyl, hydroxy, lower alkoxy, halo-lower alkoxy, cycloalkoxy or cyano, R² represents hydrogen, halogen, lower alkyl, hydroxy or lower alkoxy, or R¹ and R² together with the carbon atoms they are bound to form a ring, R³ represents hydrogen, lower alkyl, lower alkoxy-lower alkyl or halo-lower alkyl, R⁴ represents hydrogen, lower alkyl, a carbamoyl group C(O)NHR⁸, a sulfamoyl group SO₂NHR⁸, halogen or cyano, and the other substituents have the meaning described in the specification; processes for the preparation thereof, pharmaceutical compositions containing same, the use thereof optionally in combination with one or more other pharmaceutically active compounds for the therapy of neoplastic diseases and autoimmune diseases, and a method for the treatment of such a diseases.

## Description

The invention relates to novel 2-phenylsulfopyrroles, processes for the preparation thereof, pharmaceutical compositions containing same, the use thereof optionally in combination with one or more other pharmaceutically active compounds for the therapy of neoplastic diseases and autoimmune diseases, and a method for the treatment of such diseases.

### Background of the invention

Cancer is one of the leading causes of death in humans. Although a variety of drugs against neoplastic diseases have been developed and techniques are available such as surgery and radiation therapy, there is still a need for alternative and improved methods of treatment of neoplastic diseases.

Autoimmune diseases are associated with abnormal lymphoproliferation as a result of defects in the termination of lymphocyte activiation and growth. Often, such diseases are associated with inflammation like rheumatoid arthritis, insulin dependent diabetes mellitus, multiple sclerosis, systemic lupus erythematosus and the like. The treatment of such diseases is focused on anti-inflammatory and immunosuppressive drugs which in numerous cases show severe side effects. Hence, there is a need for alternative drugs with a new mode of action showing less side effects.

Apoptosis is a term used to describe a series of cellular events which occur to bring about programmed cell death. There are various apoptotic pathways, some of which have been characterized, whereas others remain to be elucidated. If the balance between cell division and apoptosis is disturbed, life-threatening diseases including cancer, autoimmune disorders, neurodegenerative and cardiovascular diseases may occur.

In recent years it has become evident that programmed cell death (apoptosis) is as important to the health of a multicellular organism as cell division. By repeated cell division and differentiation throughout development or tissue repair, surplus or even harmful cells are generated. In order to maintain tissue homeostasis these cells have to be removed or killed. The delicate interplay between cell growth and apoptosis in an organism is mirrored in the complex molecular balance that determines whether an individual cell undergoes division, arrests in the cell cycle or commits to programmed cell death.

Dysregulation of cell proliferation, or lack of appropriate cell death, has wide ranging clinical implications. A number of diseases associated with such dysregulation involve hyperproliferation, inflammation, tissue remodeling and repair. Familiar indications in this category include cancers, restenosis, neointimal hyperplasia, angiogenesis, endometriosis, lymphoproliferative disorders, transplantation related pathologies (graft rejection), polyposis, loss of neural function in the case of tissue remodeling and the like. Such cells may lose the normal regulatory control of cell division, and may also fail to undergo appropriate cell death.

As apoptosis is inhibited or delayed in most types of proliferative, neoplastic diseases, induction of apoptosis is an option for treatment of cancer, especially in cancer types which show resistance to classic chemotherapy, radiation and immunotherapy (Apoptosis and Cancer Chemotherapy, Hickman and Dive, eds., Blackwell Publishing, 1999). Also in autoimmune and transplantation related diseases and pathologies compounds inducing apoptosis may be used to restore normal cell death processes and therefore can eradicate the symptoms and might cure the diseases. Further applications of compounds inducing apoptosis may be in restenosis, i.e. accumulation of vascular smooth muscle cells in the walls of arteries, and in persistent infections caused by a failure to eradicate bacteria- and virus-infected cells. Furthermore, apoptosis can be induced or reestablished in epithelial cells, in endothelial cells, in muscle cells, and in others which have lost contact with extracellular matrix. These cells are potentially able to colonize other organs and therefore can develop into pathologies like neoplasias, endometriosis and the like.

Sulfopyrroles have been the subject of patent application PCT/IB2004/001818. It has now been found that further sulfopyrroles are selectively inducing apoptosis in cancer cells.

### Summary of the invention

2-Phenylsulfopyrroles of formula (I) are selectively inducing apoptosis in cancer cells, and can be used for the treatment of neoplastic and autoimmune diseases. The invention relates to novel compounds of formula (I) as defined hereinafter, to methods of synthesis of such compounds, to compounds of formula (I) for use as medicaments, to pharmaceutical compositions containing compounds of formula (I), to the use of a compounds of formula (I) for the preparation of a pharmaceutical composition for the treatment of neoplastic and autoimmune diseases, and to methods of treatment of neoplastic and autoimmune diseases using such compounds of formula (I) or of pharmaceutical compositions containing same.

### Detailed description of the invention

The invention relates to novel compounds of formula (I) 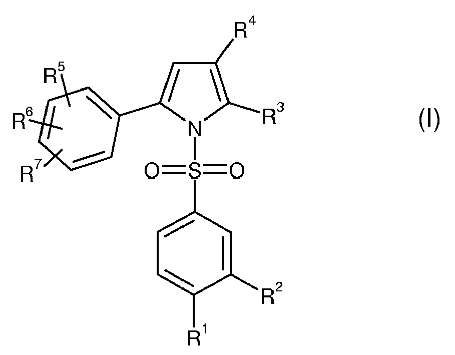 wherein
R¹ represents lower alkyl, halo-lower alkyl, cycloalkyl, hydroxy, lower alkoxy, halo-lower alkoxy, cycloalkoxy or cyano;
R² represents hydrogen, halogen, lower alkyl, hydroxy or lower alkoxy;
or R¹ and R² together with the carbon atoms they are bound to form a 5, 6 or 7 membered carbocyclic or heterocyclic ring;
R³ represents hydrogen, lower alkyl, lower alkoxy-lower alkyl or halo-lower alkyl;
R⁴ represents hydrogen, lower alkyl, a carbamoyl group C(O)NHR⁸, a sulfamoyl group SO₂NHR⁸, halogen or cyano;
R⁵, R⁶ and R⁷, independently of each other, represent hydrogen, lower alkyl, halo-lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, halo-lower alkoxy-lower alkyl, heterocyclyl, heterocyclyl-lower alkyl, optionally substituted phenyl, optionally substituted phenyl-lower alkyl, optionally substituted heteroaryl, optionally substituted heteroaryl-lower alkyl, optionally substituted alkenyl, optionally substituted alkinyl, hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy, sulfamoyloxy, carbamoyloxy, lower alkylcarbonyloxy, amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl; carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl; lower alkylcarbonyl, cycloalkylcarbonyl, optionally substituted phenylcarbonyl, optionally substituted heteroarylcarbonyl, heterocyclylcarbonyl, carboxy, lower alkoxycarbonyl, hydroxy-lower alkoxycarbonyl, lower alkoxy-lower alkoxycarbonyl, optionally substituted phenyl-lower alkoxycarbonyl, cyano, mercapto, lower alkylmercapto, optionally substituted phenylmercapto, lower alkylsulfinyl, halo-lower alkylsulfinyl, optionally substituted phenylsulfinyl, lower alkylsulfonyl, halo-lower alkylsulfonyl, optionally substituted phenylsulfonyl, optionally substituted phenyl-lower alkylsulfonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
and to compounds as defined hereinbefore for use as medicaments.

The general terms used hereinbefore and hereinafter preferably have within the context of this disclosure the following meanings, unless otherwise indicated:
The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Double bonds in principle can have E- or Z-configuration. The compounds of this invention may therefore exist as isomeric mixtures or single isomers. If not specified both isomeric forms are intended.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula (I).

Alkyl has from 1 to 12, preferably from 1 to 10 carbon atoms, and is linear or branched. Alkyl is preferably lower alkyl, for example, C₁-C₇-alkyl, preferably C₁-C₄-alkyl.

Lower alkyl has 1 to 7 carbon atoms, preferably 1 to 4 carbon atoms, and is e.g. n-hexyl, n-pentyl, butyl, such as n-butyl, sec-butyl, isobutyl or tert-butyl, propyl, such as n-propyl or isopropyl, ethyl or methyl. Most preferably lower alkyl is methyl or ethyl.

Cycloalkyl has preferably 3 to 7 ring carbon atoms, and may be unsubstitued or substituted, e.g. by lower alkyl or lower alkoxy. C₃-C₇-cycloalkyl is, for example, cyclopheptyl, cyclohexyl, cyclopentyl, methylcyclopentyl or cyclopropyl.

Aryl stands for a mono- or bicyclic fused ring aromatic group with 5 to 10 carbon atoms, such as phenyl, 1-naphthyl or 2-naphthyl, or also a partially saturated bicyclic fused ring comprising a phenyl group, such as indanyl, dihydro- or tetrahydronaphthyl. Preferably, aryl is phenyl.

In optionally substituted aryl or phenyl, substituents are preferably lower alkyl, lower alkoxy, lower alkoxy-lower alkoxy, methylenedioxy, halo-lower alkyl, lower alkoxy-lower alkyl, halo, or nitro.

Heteroaryl represents an aromatic group containing at least one heteroatom selected from nitrogen, oxygen and sulfur, and is mono- or bicyclic. Monocyclic heteroaryl includes 5 or 6 membered heteroaryl groups containing 1, 2, 3 or 4 heteroatoms selected from nitrogen, sulfur and oxygen. Bicyclic heteroaryl includes 9 or 10 membered fused-ring heteroaryl groups. Examples of heteroaryl include pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, and benzo fused derivatives of such monocyclic heteroaryl groups, such as indolyl, benzimidazolyl or benzofuryl, quinolinyl, isoquinolinyl, quinazolinyl, quinoxalinyl or purinyl, preferably pyrrolyl, thienyl, furyl, imidazolyl, triazolyl, oxazolyl, pyridyl, pyrimidinyl or pyrazinyl.

In optionally substituted heteroaryl, substituents are preferably lower alkyl, halo-lower alkyl, lower alkoxy-lower alkyl, lower alkoxy, lower alkoxy-lower alkoxy, amino optionally substituted by one or two substituents lower alkyl and one substituent lower alkylcarbonyl, halo, or nitro.

Alkenyl contains one or more, e.g. two or three, double bonds, and is preferably lower alkenyl, such as C₁-C₄-alkenyl, e.g. 1- or 2-butenyl, 1-propenyl, allyl or vinyl.

Alkinyl is preferably lower alkinyl, such as C₁-C₄-alkinyl, e.g. propargyl or acetylenyl.

In optionally substituted alkenyl or alkinyl, substituents are preferably lower alkyl, lower alkoxy, halo or di(lower alkyl)amino, and are connected with a saturated carbon atom of alkenyl or alkinyl or with an unsaturated carbon atom of alkenyl.

Heterocyclyl designates preferably a saturated, partially saturated or unsaturated, mono- or bicyclic ring containg 4-10 atoms comprising one, two or three heteroatoms selected from nitrogen, oxygen and sulfur, which may, unless otherwise specified, be carbon or nitrogen linked, wherein a ring nitrogen atom may optionally be substituted by a group selected from lower alkyl, amino-lower alkyl, aryl, aryl-lower alkyl and acyl, and a ring carbon atom may be substituted by lower alkyl, amino-lower alkyl, aryl, aryl-lower alkyl, heteroaryl, lower alkoxy, hydroxy or oxo. Examples of heterocyclyl are pyrrolidinyl, oxazolidinyl, thiazolidinyl, piperidinyl, morpholinyl, piperazinyl, dioxolanyl and tetrahydropyranyl.

Acyl designates, for example, alkylcarbonyl, cyclohexylcarbonyl, arylcarbonyl, aryl-lower alkylcarbonyl, or heteroarylcarbonyl. Lower acyl is preferably C₁-C₄-alkylcarbonyl, in particular propionyl or acetyl.

Hydroxyalkyl is especially hydroxy-lower alkyl, preferably hydroxy-C₁-C₄-alkyl, e.g. hydroxymethyl, 2-hydroxyethyl or 2-hydroxy-2-propyl.

Haloalkyl is especially halo-lower alkyl, preferably halo-C₁-C₄-alkyl, e.g. fluoroalkyl, especially trifluoromethyl, pentafluoroethyl or 3,3,3-trifluoroethyl.

Halogen is fluorine, chlorine, bromine or iodine.

Lower alkoxy is especially C₁-C₄-alkoxy, e.g. methoxy, ethoxy, isopropyloxy, or tert-butyloxy.

Arylalkyl includes aryl and alkyl as defined hereinbefore, and is e.g. benzyl, 1-phenethyl or 2-phenethyl.

Heteroarylalkyl includes heteroaryl and alkyl as defined hereinbefore, and is e.g. 2-pyridyl-methyl, 3-pyridyl-methyl, 4-pyridyl-methyl, 1- or 2-pyrrolyl-methyl, 1-imidazolyl-methyl, 2-(1-imidazolyl)-ethyl or 3-(1-imidazolyl)-propyl.

Two adjacent substituents which together with the atoms of aryl or heteroaryl may form a 5, 6 or 7 membered carbocyclic or heterocyclic ring are, for example, propylene, 1- or 2-oxopropylene, 1- or 2-oxapropylene, 1-oxapropylidene, methylenedioxy, difluoro-methylenedioxy, 1- or 2-azapropylene, 1- or 2-azapropylidene, 1,2- or 1,3-diaza-propylidene, 1,3-diaza-2-oxopropylene, butylene, 1- or 2-oxabutylene, ethylenedioxy, 1- or 2-azabutylene, or 1- or 2-azabutadienylidene, pentylene, 1-, 2- or 3-oxapentylene, propylenedioxy, 1-, 2- or 3-azapentylene, or such groups carrying further substituents as defined hereinbefore, e.g. lower alkyl, such as methyl.

In substituted amino, the substituents are preferably lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl or lower alkylcarbonyl, or the two substituents on nitrogen form together with the nitrogen heteroaryl or heterocyclyl. In particular, substituted amino is C₁-C₄-alkylamino, di-C₁-C₄-alkylamino, optionally substituted phenylamino or optionally substituted phenyl-C₁-C₄-alkylamino.

Substituents R⁵, R⁶ and R⁷ may be located in any position of the phenyl ring. Preferred are such substituents in position 2, 3 and 4, or 2, 4 and 5, or 2, 4 and 6, and in particular in position 3, 4 and 5.

Salts are especially the pharmaceutically acceptable salts of compounds of formula (I).

Such salts are formed, for example, as acid addition salts, preferably with organic or inorganic acids, from compounds of formula (I) with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantine-carboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

The compound of the formula (I) may be administered in the form of a pro-drug which is broken down in the human or animal body to give a compound of the formula (I). Examples of pro-drugs include in vivo hydrolysable esters of a compound of the formula (I).

The compounds of formula (I) have valuable pharmacological properties. The invention also relates to compounds of formula (I) as defined hereinbefore for use as medicaments.

The efficacy of the compounds of the invention in inducing apoptosis in tumor cells can be demonstrated as follows:
Relative fluorescent activities of suitable tumor cell lines transfected with green fluorescent protein (GFP) are measured in the presence of compounds of the invention and of standard tumor drugs, using the method described in WO 99/35493. Suitable tumor cell lines are A20.2J, a BALB/c B cell lymphoma, PB-3c, an IL-3 dependent, non tumorigenic mastocyte line isolated from the bone marrow of a DBA/2 mouse, Jurkat, a human acute T cell leukemia cell line, K562, a human chronic myelogenous leukemia cell line, HL60, a human acute promyelocytic leukemia cell line, Ramos and Raji, human B-cell lymphoma cell lines, H9 and Hut78, human T-cell lymphoma cell lines, HeLa and KB, human squamous cell carcinoma cell lines, MCF7, SK-BR-3, PC3, HBL-100, SW480, H460 and H1792, human adenocarcinoma cell lines and HT-1 080, a human fibrosarcoma cell line.

Preferred standard drugs as compounds for comparisons are: a) antimetabolites such as 5-fluorouracil (ICN), gemcitabine HCl (Gemzar™, Eli Lilly), b) alkylating agents such as oxaliplatin (Eloxantin™, Sanofi-Synthélabo), dacarbazin (Detimedac™, Medac), cyclophosphamide (Endoxan™, Asta) and carboplatin (Paraplatin™, Bristol-Meyers Squibb), c) cell-cycle inhibitor such as vinorelbine (Navelbine™, Robapharm), vinblastine (Velbe™, Eli Lilly), docetaxel (Taxotere™, Aventis), d) DNA breaker (topo-isomerase inhibitor, intercalator, strand breaker) such as doxorubicin HCl (Adriblastin™, Pharmacia-Upjohn), bleomycin (Asta-Medica), irinotecan (Campto™, Aventis), etoposide phosphate (Etopophos™, Bristol-Meyers Squibb), topotecan HCl, (Hycamtin™, GlaxoSmithKline), e) mixtures thereof, f) compounds interfering with the signal transduction pathway, such as caspase activity modifiers, agonists and antagonists of cell death receptors, modifiers of nucleases, phosphatases and kinases such as imatinib mesylate (Gleevec™, Novartis), dexamethasone, phorbol myristate acetate, cyclosporin A, quercetin, tamoxifen (Alexis Corporation, Switzerland).

Apoptosis is determined in a primary screen using a fluorescence plate reader and then in a secondary screen using FACS (fluorescence activated cell scanning). Compounds causing apoptosis without substantial cytotoxic side effects are chosen for further testing and characterization by using a combination of the following well established assays:
A) Nuclear staining with Hoechst 33342 dye providing information about nuclear morphology and DNA fragmentation which are hallmarks of apoptosis. B) MTS proliferation assay measuring the metabolic activity of cells. Viable cells are metabolically active whereas cells with compromised respiratory chain show a reduced activity in this test. C) AnnexinV binding assay which reflects the phosphatidylserine content of the outer lipid bilayer of the plasma membrane. This event is considered an early hallmark of apoptosis. D) PI staining for cell cycle distribution which shows any alterations in the distribution among the different phases of the cell cycle. Cell cycle arresting points can be determined. E) Proliferation assay monitoring DNA synthesis by incorporating bromodeoxyuridine (BrdU). Inhibitory effects on growth/proliferation can be directly determined. F) Cystein proteinase dependency, respectively caspase dependency are determined by using specific inhibitors. This provides information about possible involvement of specific proteases in the mechanisms.

On the basis of these studies, a compound of formula (I) according to the invention shows therapeutic efficacy especially against neoplastic diseases and autoimmune diseases. In particular, the compounds of the invention are active against malignancies, e.g. epithelial neoplasms, squamous cell neoplasms, basal cell neoplasms, transitional cell papillomas and carcinomas, adenomas und adenocarcinomas, adnexal and skin appendage neoplasms, mucoepidermoid neoplasms, cystic neoplasms, mucinous and serous neoplasms, ductal-, lobular and medullary neoplasms, acinar cell neoplasms, complex epithelial neoplasms, specialized gonadal neoplasms, paragangliomas and glomus tumors, naevi and melanomas, soft tissue tumors and sarcomas, fibromatous neoplasms, myxomatous neoplasms, lipomatous neoplasms, myomatous neoplasms, complex mixed and stromal neoplasms, fibroepithelial neoplasms, synovial like neoplasms, mesothelial neoplasms, germ cell neoplasms, trophoblastic neoplasms, mesonephromas, blood vessel tumors, lymphatic vessel tumors, osseous and chondromatous neoplasms, giant cell tumors, miscellaneous bone tumors, odontogenic tumors, gliomas, neuro-epitheliomatous neoplasms, meningiomas, nerve sheath tumors, granular cell tumors and alveolar soft part sarcomas, Hodgkin's and non Hodgkin's lymphomas, other lympho-reticular neoplasms, plasma cell tumors, mast cell tumors, immunoproliferative diseases, leukemias, miscellaneous myeloproliferative disorders, lymphoproliferative disorders and myelodysplastic syndromes.

The compounds of the invention are likewise active against autoimmune diseases, e.g. against systemic, discoid or subacute cutaneous lupus erythematosus, rheumatoid arthritis, antiphospholipid syndrome, CREST, progressive systemic sclerosis, mixed connective tissue disease (Sharp syndrome), Reiter's syndrome, juvenile arthritis, cold agglutinin disease, essential mixed cryoglobulinemia, rheumatic fever, ankylosing spondylitis, chronic polyarthritis, myasthenia gravis, multiple sclerosis, chronic inflammatory demyelinating polyneuropathy, Guillan-Barré syndrome, dermatomyositis/polymyositis, autoimmune hemolytic anemia, thrompocytopenic purpura, neutropenia, type I diabetes mellitus, thyroiditis (including Hashimoto's and Grave' disease), Addison's disease, polyglandular syndrome, pemphigus (vulgaris, foliaceus, sebaceous and vegetans), bullous and cicatricial pemphigoid, pemphigoid gestationis, epidermolysis bullosa acquisita, linear IgA disease, lichen sclerosus et atrophicus, morbus Duhring, psoriasis vulgaris, guttate, generalized pustular and localized pustular psoriasis, vitiligo, alopecia areata, primary biliary cirrhosis, autoimmune hepatitis, all forms of glomerulonephritis, pulmonal hemorrhage (goodpasture syndrome), IgA nephropathy, pernicious anemia and autoimmune gastritis, inflammatory bowel diseases (including colitis ulcerosa and morbus Crohn), Behcet's disease, Celic-Sprue disease, autoimmune uveitis, autoimmune myocarditis, granulomatous orchitis, aspermatogenesis without orchitis, idiopatic and secondary pulmonary fibrosis, inflammatory diesases with a possibility of autoimmune pathogensesis, such as pyoderma gangrensosum, lichen ruber, sarcoidosis (including Löfgren and cutaneous/subcutaneous type), granuloma anulare, allergic type I and type IV immunolgical reaction, asthma bronchiale, pollinosis, atopic, contact and airborne dermatitis, large vessel vasculitis (giant cell and Takayasu's arteritis), medium sized vessel vasculitis (polyarteritis nodosa, Kawasaki disease), small vessel vasculitis (Wegener's granulomatosis, Churg Strauss syndrome, microscopic polangiitis, Henoch-Schoenlein purpura, essential cryoglobulinemic vasculitis, cutaneous leukoklastic angiitis), hypersensitivity syndromes, toxic epidermal necrolysis (Stevens-Johnson syndrome, erythema multiforme), diseases due to drug side effects, all forms of cutaneous, organ-specific and systemic effects due to type I-VI (Coombs classification) immunologic forms of reaction, transplantation related pathologies, such as acute and chronic graft versus host and host versus graft disease, involving all organs (skin, heart, kidney, bone marrow, eye, liver, spleen, lung, muscle, central and peripheral nerve system, connective tissue, bone, blood and lymphatic vessel, genito-urinary system, ear, cartillage, primary and secondary lymphatic system including bone marrow, lymph node, thymus, gastrointestinal tract, including oro-pharynx, esophageus, stomach, small intestine, colon, and rectum, including parts of above mentioned organs down to single cell level and substructures, e.g. stem cells).

A compound of formula (I) can be administered alone or in combination with one or more other therapeutic agents, possible combination therapy taking the form of fixed combinations, or the administration of a compound of the invention and one or more other therapeutic agents being staggered or given independently of one another, or the combined administration of fixed combinations and one or more other therapeutic agents. A compound of formula (I) can, besides or in addition, be administered especially for tumor therapy in combination with chemotherapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk. Particularly preferred is the use of compounds of formula (I) in combination with radiotherapy.

Therapeutic agents for possible combination are especially one or more cytostatic or cytotoxic compounds, for example a chemotherapeutic agent or several selected from the group comprising indarubicin, cytarabine, interferon, hydroxyurea, bisulfan, or an inhibitor of polyamine biosynthesis, an inhibitor of protein kinase, especially of serine/threonine protein kinase, such as protein kinase C, or of tyrosine protein kinase, such as epidermal growth factor receptor tyrosine kinase, a cytokine, a negative growth regulator, such as TGF-β or IFN-β, an aromatase inhibitor, a classical cytostatic, an inhibitor of the interaction of an SH2 domain with a phosphorylated protein, an inhibitor of Bcl-2 and modulators of the Bcl-2 family members such as Bax, Bid, Bad, Bim, Nip3 and BH3-only proteins.

A compound according to the invention is not only for the (prophylactic and preferably therapeutic) management of humans, but also for the treatment of other warm-blooded animals, for example of commercially useful animals, for example rodents, such as mice, rabbits or rats, or guinea-pigs. Such a compound may also be used as a reference standard in the test systems described above to permit a comparison with other compounds.

With the groups of preferred compounds of formula (I) mentioned hereinafter, definitions of substituents from the general definitions mentioned hereinbefore may reasonably be used, for example, to replace more general definitions with more specific definitions or especially with definitions characterized as being preferred.

In particular, the invention refers to novel compounds of formula (I) wherein
R¹ represents lower alkyl, cycloalkyl, hydroxy, lower alkoxy, cycloalkoxy or cyano;
R² represents hydrogen;
R³ represents hydrogen, lower alkyl, lower alkoxy-lower alkyl or halo-lower alkyl;
R⁴ represents hydrogen, lower alkyl, a carbamoyl group C(O)NHR⁸, a sulfamoyl group SO₂NHR⁸, halogen or cyano;

R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, heterocyclyl-lower alkyl, optionally substituted phenyl, optionally substituted phenyl-lower alkyl, optionally substituted heteroaryl, optionally substituted heteroaryl-lower alkyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower
alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl;
lower alkylcarbonyl, cycloalkylcarbonyl, optionally substituted phenylcarbonyl, optionally substituted heteroarylcarbonyl, heterocyclylcarbonyl,
carboxy, lower alkoxycarbonyl, hydroxy-lower alkoxycarbonyl, lower alkoxy-lower alkoxycarbonyl, optionally substituted phenyl-lower alkoxycarbonyl, cyano,
mercapto, lower alkylmercapto, optionally substituted phenylmercapto, lower alkylsulfinyl, halo-lower alkylsulfinyl, optionally substituted phenylsulfinyl, lower alkylsulfonyl, halo-lower alkylsulfonyl, optionally substituted phenylsulfonyl, optionally substituted phenyl-lower alkylsulfonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;

R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
and to such compounds for use as medicaments.

More particularly, the invention refers to novel compounds of formula (I) wherein
R¹ represents lower alkyl, cycloalkyl, hydroxy, lower alkoxy, cycloalkoxy or cyano;
R² represents hydrogen;
R³ represents hydrogen, lower alkyl or lower alkoxy-lower alkyl;
R⁴ represents hydrogen, lower alkyl, a carbamoyl group C(O)NHR⁸, a sulfamoyl group SO₂NHR⁸ or cyano;
R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, optionally substituted heteroaryl, optionally substituted heteroaryl-lower alkyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower
alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl;
lower alkylcarbonyl, cycloalkylcarbonyl, optionally substituted phenylcarbonyl, optionally substituted heteroarylcarbonyl, heterocyclylcarbonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;

R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
and to such compounds for use as medicaments.

More preferably, the invention refers to compounds of formula (I) wherein
R¹ represents lower alkyl, hydroxy or lower alkoxy;
R² represents hydrogen;
R³ represents hydrogen, lower alkyl or lower alkoxy-lower alkyl;
R⁴ represents hydrogen, lower alkyl, a carbamoyl group C(O)NHR⁸ or cyano;

R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower
alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
and to such compounds as defined hereinbefore for use as medicaments.

Still more preferably, the invention refers to novel compounds of formula (I) wherein
R¹ represents hydroxy or lower alkoxy;
R² represents hydrogen;
R³ represents hydrogen, lower alkyl or lower alkoxy-lower alkyl;
R⁴ represents hydrogen, lower alkyl, a carbamoyl group C(O)NHR⁸ or cyano;

R⁵, R⁶ and R⁷, independently of each other, represent hydrogen, lower alkyl, lower alkoxy-lower alkyl, hydroxy, lower alkoxy, cycloalkoxy, hydroxy-lower alkoxy, lower alkoxy-lower alkoxy, amino, amino-lower alkyl or amino-lower alkylamino, wherein in each case the nitrogen atom is unsubstituted or substituted by one or two substitutents selected from lower alkyl, hydroxy-lower alkyl, lower alkoxy-lower alkyl, hydroxy-lower alkoxy-lower alkyl, lower alkylcarbonyl, lower alkoxycarbonyl and lower aminocarbonyl, or wherein the two substituents on nitrogen form together with the nitrogen heteroaryl or heterocyclyl; halogen, and cyano;
with the proviso that at least one of R⁵, R⁶ and R⁷ is different from hydrogen;

R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl; optionally substituted phenyl, optionally substituted phenyl-lower alkyl, optionally substituted heteroaryl, optionally substituted heteroaryl-lower alkyl, or heterocyclyl;
and salts thereof;
and to such compounds as defined hereinbefore for use as medicaments.

Most preferred are the compounds of the Examples, especially the compounds of Examples 2 and 3, for use as medicaments.

Especially, the invention relates to the use of a compound of formula (I), a prodrug or a pharmaceutically acceptable salt of such a compound for the preparation of a pharmaceutical composition for the treatment of a neoplastic disease, autoimmune disease, transplantation related pathology and/or degenerative disease.

Furthermore, the invention provides a method for the treatment of a neoplastic disease, autoimmune disease, transplantation related pathology and/or degenerative disease, which comprises administering a compound of formula (I), a prodrug or a pharmaceutically acceptable salt thereof, wherein the radicals and symbols have the meanings as defined above, in a quantity effective against said disease, to a warm-blooded animal requiring such treatment.

### Method of preparation

A compound of the invention may be prepared by processes that, though not applied hitherto for the new compounds of the present invention, are known *per se,* in particular
A) a process, wherein a pyrrole of formula (II) 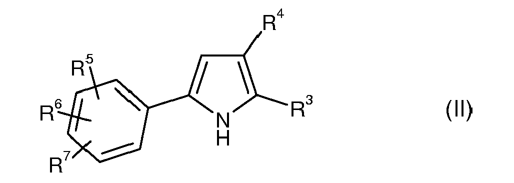 is reacted with a sulfonyl chloride of formula (III) 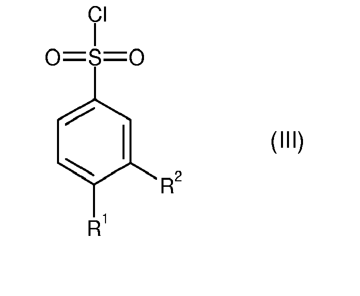 wherein the substituents are as defined for a compound of formula (I), or a derivative thereof wherein functional groups are in protected form, in the presence of a strong base and an inert solvent, and any protecting groups in a protected derivative of a compound of the formula (I) are removed;
B) a process, wherein a sulfonamide of formula (IV) 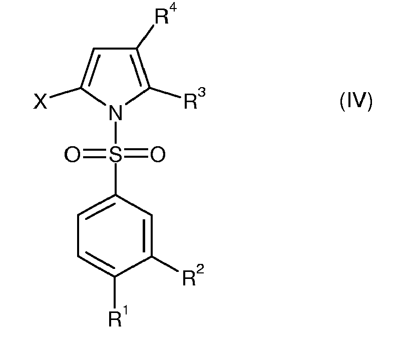 is reacted with a compound of formula (V) 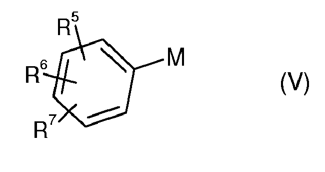 wherein the substituents R¹ to R⁷ are as defined for a compound of formula (I), X represents halogen and M represents alkali metal, substituted silicon or boronic acid, or a derivative thereof wherein functional groups are in protected form, and any protecting groups in a protected derivative of a compound of the formula (I) are removed;
C) a process, wherein a sulfonamide of formula (VI) 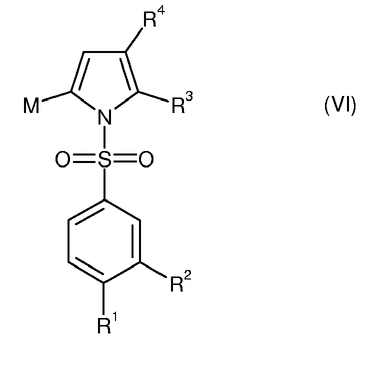 is reacted with a compound of formula (VII) 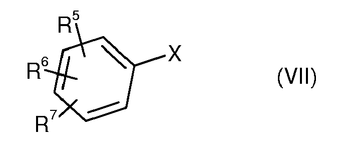 wherein the substituents R¹ to R⁷ are as defined for a compound of formula (I), X represents halogen and M represents alkali metal, substituted silicon or boronic acid, or a derivative thereof wherein functional groups are in protected form, and any protecting groups in a protected derivative of a compound of the formula (I) are removed; or
D) for the preparation of a compound of formula (I) wherein R³ is hydrogen and R⁴ is chlorine or bromine, a process wherein a benzoyl acetylene of formula (VIII) 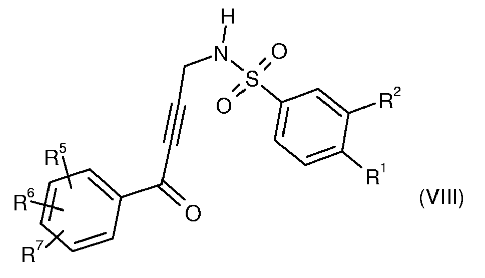 wherein R¹, R², R⁵, R⁶ and R⁷ are as defined for a compound of formula (I), or a derivative thereof wherein functional groups are in protected form, is reacted with hydrochloric or hydrobromic; or
E) for the preparation of a compound of formula (I) wherein R⁴ represents a carbamoyl group C(O)NHR⁸ or a sulfamoyl group SO₂NHR⁸, a process wherein a compound of formula (I) wherein R⁴ is a carboxylic acid or sulfonic acid residue or the corresponding acid halide, or a derivative thereof wherein functional groups are in protected form, is reacted with an amine of formula (IX)

   R⁸NH₂ (IX)

   wherein R⁸ has the meaning as defined under formula (I), or a derivative thereof wherein functional groups are in protected form;
   and, if so desired, an obtainable compound of formula (I) is converted into another compound of formula (I), a free compound of formula (I) is converted into a salt, an obtainable salt of a compound of formula (I) is converted into the free compound or another salt, and/or a mixture of isomeric compounds of formula (I) is separated into the individual isomers.

A strong base to be used in the sulfonylation of a pyrrole of formula (II) according to process A) may be a metal hydride such as sodium hydride, a metal amide such as lithium diisopropylamide, a phosphazene base such as Verkade's superbase (2,8,9-trimethyl-2,5,8,9-tetraaza-1-phosphabicyclo[3.3.3]undecane), or also an inorganic solid base only partially soluble, e.g. potassium hydroxide or potassium carbonate, preferably under phase transfer conditions. The reaction can be carried out in a manner known per se, usually in the presence of a suitable solvent, e.g. an excess of the tertiary amine base, an inert polar solvent such as pyridine or a dipolar aprotic solvent such as dimethyl formamide or the like, with cooling or heating, for example in a temperature range from approximately -30°C to approximately +150°C, especially approximately around room temperature.

The coupling of a halide of formula (IV) or (VIII) with the corresponding metallated compound of formula (V) or (VI) according to process B) or C), respectively, is performed under standard conditions known per se. Suitable halogens X are chlorine, bromine or iodine. Metals M are e.g. alkali metals, preferably lithium or magnesium (Grignard reagent). The particular reaction of a pyrrole of formula (VI) metallated with magnesium with an iodinated phenyl derivative of formula (VII) may be performed in analogy to the reaction described by A. Dinsmore et al., Organic Letters 6, 293-296 (2004).

The reaction of a bromo or iodo substituted pyrrole of formula (IV) with a suitable boronic acid of formula (V) or the corresponding boronic acid ester is known as Suzuki reaction, and is preferably executed under conditions known *per se* for a Suzuki reaction, for example as described by L.W. Knight et al., Synlett 2003 (13), 1993-1996. Likewise the reaction of a pyrrole boronic acid of formula (VI) or the corresponding boronic acid ester, e.g. the cylic ester with 2,2-dimethylpropane-1,3-diol, with the corresponding halide of formula (VII) is performed. Preferably a dipolar aprotic solvent such as dimethyl formamide, or a polar ether, e.g. dioxane, tetrahydrofuran or dimethoxyethane, is used, and the reaction is performed with moderate heating, for example in a temperature range from approximately +30°C to approximately +150°C, especially approximately around 40°C to 80°C, in the presence of a soluble palladium(0) or related metal catalyst, for example tetrakis(triphenylphosphine)palladium. Alternatively, the reaction may also be performed with a compound of formula (IV) or (VII) wherein X is chlorine. Further catalysts considered are tetrakis(trimethoxyphosphine)palladium, tris(dibenzylideneacetone)-palladium or dichlorobis(triphenylphosphine)palladium(II), and also nickel catalysts, e.g. dichlorobis(triethylamine)nickel(II). The reaction is preferably performed in the presence of a base, e.g solid or aqueous sodium or potassium carbonate, potassium hydroxide or potassium phosphate.

Ring closure of the acetylenic ketone of formula (VIII) in process D) is performed with hydrochloric or hydrobromic acid und reaction conditions known per se, e.g. as described in T. Masquelin and D. Obrecht, Synthesis 1995, 276-284.

Amidation of a carboxylic or sulfonic acid or the corresponding carbonyl or sufonyl halides with an amine of formula (IX) in process E) is performed under reaction conditions known per se. For the formation of a carboxamide, conditions and reagents generally used for peptide coupling may be applied, for example using a carbodiimide derivative, a suitable hydroxyimide, 1-hydroxybenztriazole or the like as condensing agent. The amidation of a sulfonyl chloride may be performed under conditions described for process A).

If one or more other functional groups, for example carboxy, hydroxy or amino, are or need to be protected in a compound of formulas (II), (III), (IV), (V), (VI), (VII), (VIII) and (IX), because they should not take part in the reaction, these are such protecting groups as are usually applied in the synthesis of amides, in particular peptide compounds, cephalosporins, penicillins, nucleic acid derivatives and sugars.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end products. The specialist knows, or can easily establish, which protecting groups are suitable with the reactions mentioned hereinabove and hereinafter.

The protection of such functional groups by such protecting groups, the protecting groups themselves, and their removal reactions are described for example in standard reference books for peptide synthesis and in special books on protective groups such as J. F. W. McOmie, "Protective Groups in Organic Chemistry", Plenum Press, London and New York 1973, in "Methoden der organischen Chemie" (Methods of organic chemistry), Houben-Weyl, 4th edition, Volume 15/l, Georg Thieme Verlag, Stuttgart 1974, and in T. W. Greene, "Protective Groups in Organic Synthesis", Wiley, New York.

In the additional process steps, carried out as desired, functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

In the conversion of an obtainable compound of formula (I) into another compound of formula (I), an amino group may be alkylated or acylated to give the correspondingly substituted compounds. Alkylation may be performed with an alkyl halide or an activated alkyl ester. For methylation, diazomethane may be used. Alkylation may also be performed with an aldehyde under reducing conditions. For acylation the corresponding acyl chloride is preferred. Alternatively, an acid anhydride may be used, or acylation may be accomplished with the free acid under conditions used for amide formation known *per se* in peptide chemistry, e.g. with activating agents for the carboxy group, such as 1-hydroxybenzotriazole, optionally in the presence of suitable catalysts or co-reagents.

Reduction of a nitro group in a nitro-substituted aryl or heteroaryl group to give the corresponding amino group is done, e.g., with iron powder in alcohol or with other reducing agents.

A carboxy group in a carboxy-substituted aryl or heteroaryl group may be amidated under conditions used for amide formation known *per se* in peptide chemistry, e.g. with the corresponding amine and an activating agent for the carboxy group, such as 1-hydroxybenzotriazole, optionally in the presence of suitable catalysts or co-reagents.

A bromo or iodo substitutent in an aryl or heteroaryl group may be replaced by phenyl or a phenyl derivative by reaction with a suitable phenylboronic acid in a Suzuki reaction, preferably in a dipolar aprotic solvent such as dimethyl formamide, or in a polar ether, e.g. tetrahydrofuran or dimethoxyethane, in the presence of a soluble palladium(0) or related metal catalyst, for example tetrakis(triphenylphosphine)palladium.

Salts of a compound of formula (I) with a salt-forming group may be prepared in a manner known *per se.* Acid addition salts of compounds of formula (I) may thus be obtained by treatment with an acid or with a suitable anion exchange reagent.

Salts can usually be converted to free compounds, e.g. by treating with suitable basic agents, for example with alkali metal carbonates, alkali metal hydrogencarbonates, or alkali metal hydroxides, typically potassium carbonate or sodium hydroxide.

It should be emphasized that reactions analogous to the conversions mentioned in this chapter may also take place at the level of appropriate intermediates.

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralising agents, for example ion exchangers, typically cation exchangers, for example in the H⁺ form, depending on the type of reaction and/or reactants at reduced, normal, or elevated temperature, for example in the range from -100°C to about 190°C, preferably from about -80 °C to about 150 °C, for example at -80 to +60 °C, at -20 to +40 °C, at room temperature, or at the boiling point of the solvent used, under atmospheric pressure or in a closed vessel, where appropriate under pressure, and/or in an inert atmosphere, for example under argon or nitrogen.

Salts may be present in all starting compounds and transients, if these contain salt-forming groups. Salts may also be present during the reaction of such compounds, provided the reaction is not thereby disturbed.

At all reaction stages, isomeric mixtures that occur can be separated into their individual isomers, e.g. diastereomers or enantiomers, or into any mixtures of isomers, e.g. racemates or diastereomeric mixtures.

The invention relates also to those forms of the process in which one starts from a compound obtainable at any stage as a transient and carries out the missing steps, or breaks off the process at any stage, or forms a starting material under the reaction conditions, or uses said starting material in the form of a reactive derivative or salt, or produces a compound obtainable by means of the process according to the invention and further processes the said compound *in situ.* In the preferred embodiment, one starts from those starting materials which lead to the compounds described hereinabove as preferred, particularly as especially preferred, primarily preferred, and/or preferred above all.

In the preferred embodiment, a compound of formula (I) is prepared according to or in analogy to the processes and process steps defined in the Examples.

The compounds of formula (I), including their salts, are also obtainable in the form of hydrates, or their crystals can include for example the solvent used for crystallization, i.e. be present as solvates.

New starting materials and/or intermediates, as well as processes for the preparation thereof, are likewise the subject of this invention. In the preferred embodiment, such starting materials are used and reaction conditions so selected as to enable the preferred compounds to be obtained.

Starting materials of formula (II), (III), (IV), (V), (VI), (VII), (VIII) or (IX) are known, commercially available, or can be synthesized in analogy to or according to methods that are known in the art. Starting compounds of formula (I) wherein R⁴ is a carboxylic acid or sulfonic acid residue or the corresponding acid halides are prepared in analogy to the processes A) to D) described above.

Pyrroles of formula (II) are obtainable in a Paal-Knorr synthesis from the corresponding 1,4-diketones with ammonia, e.g. in the form of ammonium carbonate. The corresponding 1,4-diketones may be synthesized from acetoacetates and a haloketone according to standard procedures.

2-Halopyrroles of formula (IV) are obtained by halogenation of pyrrole and subsequent reaction with a sulfonyl chloride of formula (III). 2-lodopyrroles are obtainable from the corresponding magnesiated pyrroles of formula (VI) (M = Mg) with iodine according to A. Dinsmore et al., Organic Letters 6, 293-296 (2004). Other 2-metallated pyrroles of formula (VI) may be formed by metal exchange reactions of magnesiated pyrroles, by reaction of 2-iodopyrroles with butyllithium or isopropylmagnesium chloride, optionally in the presence of lithiumchloride, or by direct lithiation of the corresponding pyrrole by butyllithium or lithium diisopropyl amide. A 2-lithiated pyrrole of formula (VI) may then be further reacted with tri-isopropoxyborate followed by hydrolysis to give the corresponding boronic acid.

Acetylenic ketones of formula (VIII) may be synthesized following the procedure of D. Obrecht, Helv. Chim. Acta 72, 447 (1989).

### Pharmaceutical preparations, methods, and uses

The present invention relates also to pharmaceutical compositions that comprise a compound of formula (I) as active ingredient and that can be used especially in the treatment of the diseases mentioned at the beginning. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions comprise the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention relates especially to pharmaceutical compositions that comprise a compound of formula (I), a tautomer, a prodrug or a pharmaceutically acceptable salt, or a hydrate or solvate thereof, and at least one pharmaceutically acceptable carrier.

The invention relates also to pharmaceutical compositions for use in a method for the prophylactic or especially therapeutic management of the human or animal body, in particular in a method of treating neoplastic disease, autoimmune disease, transplantation related pathology and/or degenerative disease, especially those mentioned hereinabove.

The invention relates also to processes and to the use of compounds of formula (I) thereof for the preparation of pharmaceutical preparations which comprise compounds of formula (I) as active component (active ingredient).

A pharmaceutical composition for the prophylactic or especially therapeutic management of a neoplastic disease, autoimmune disease, transplantation related pathology and/or degenerative disease, of a warm-blooded animal, especially a human or a commercially useful mammal requiring such treatment, comprising a novel compound of formula (I) as active ingredient in a quantity that is prophylactically or especially therapeutically active against the said diseases, is likewise preferred.

The pharmaceutical compositions comprise from approximately 1% to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

The pharmaceutical compositions of the present invention are prepared in a manner known *per se,* for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se,* for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80^{□} (polyoxyethylene(20)sorbitan mono-oleate).

Suspensions in oil comprise as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol. As mixtures of fatty acid esters, vegetable oils such as cottonseed oil, almond oil, olive oil, castor oil, sesame oil, soybean oil and groundnut oil are especially useful.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

The present invention relates furthermore to a method for the treatment of a neoplastic disease, autoimmune disease, transplantation related pathology and/or degenerative disease, which comprises administering a compound of formula (I) or a pharmaceutically acceptable salt thereof, wherein the radicals and symbols have the meanings as defined above for formula (I), in a quantity effective against said disease, to a warm-blooded animal requiring such treatment. The compounds of formula (I) can be administered as such or especially in the form of pharmaceutical compositions, prophylactically or therapeutically, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

The present invention relates especially also to the use of a compound of formula (I), or a pharmaceutically acceptable salt thereof, especially a compound of formula (I) which is said to be preferred, or a pharmaceutically acceptable salt thereof, as such or in the form of a pharmaceutical formulation with at least one pharmaceutically acceptable carrier for the therapeutic and also prophylactic management of one or more of the diseases mentioned hereinabove, in particular a neoplastic disease, autoimmune disease, transplantation related pathology and/or degenerative disease.

The preferred dose quantity, composition, and preparation of pharmaceutical formulations (medicines) which are to be used in each case are described above.

The following Examples serve to illustrate the invention without limiting the invention in its scope.

### Examples

Abbreviations: eq. = equivalent(s); MS = mass spectrum; THF = tetrahydrofuran. Analytical HPLC retention times (Rₜ in minutes) are determined using a Develosil RP Aqueous 4.6x 50mm column No. 1610101 (Phenomenex) with the following gradients:
program 3: 30% CH₃CN; 70% H₂O + 0.1 % trifluoroacetic acid; flow 1.5 ml/min for 2 min; within 5 min to 100%CH₃CN;
program 4: 40% CH₃CN; 60% H₂O + 0.1 % trifluoroacetic acid; flow 1.5 ml/min for 2 min; within 5 min to 100%CH₃CN;

### Example 1: 4-Bromo-1-p-methoxyphenylsulfonyl-2-(3,4,5-trimethoxyphenyl)-pyrrole

To a stirred solution of 3-p-methoxyphenylsulfonylamino-1-propinyl 3,4,5-trimethoxyphenyl ketone (2.02 g, 2.78 mmol, Example 1a) in CH₂Cl₂ (10 ml) a solution of HBr (732 µl, 1.5 equiv., 33% in acetic acid) is added at 4°C. The reaction mixture is stirred for 45 min at 4°C, and poured onto ice, saturated aqueous NaHCO₃ solution and ethyl acetate. The organic layer is dried (MgSO₄), the solvents are evaporated and the residue chromatographed on SiO₂ with ethyl acetate/hexane (1:3) to yield the title compound (2.0 g, 86.3%) as a pink solid. ¹H-NMR (300 MHz, DMSO-d₆): 7.65 (d, J=1.9, 1 H); 7.38 (d, J=9.0, 2H); 7.02 (d, J=9.0, 2H); 6.40 (m, 3H); 3.82 (s, MeO); 3.72 (s, MeO); 3.69 (s, 2MeO).

### Example 1a: 3-p-Methoxyphenylsulfonylamino-1-propinyl 3,4,5-trimethoxyphenyl ketone

To a stirred solution of propargyl p-methoxyphenylsulfonylamide (1.13 g, 5.0 mmol, Example 1b) in dry THF (15 ml) lithium diisopropylamide solution (5.5 ml, 2.2 equiv., 2M in THF) is added under argon at -78°C. The reaction mixture is stirred for 1 h at -78°C followed by addition of 3,4,5-trimethoxybenzaldehyde (1.15 g, 6 mmol, 2 equiv.), then again stirred for 30 min at -78°C and allowed to come to 4°C. After stirring for 1 h at 4°C and for 1 h at room temperature, the reaction mixture is poured onto ice, saturated ammonium chloride solution and ethyl acetate. The organic layer is extracted with brine, dried (MgSO₄), the solvents are evaporated and the residue chromatographed on SiO₂ with ethyl acetate/hexane (1:1 to 2:1) to yield the intermediate alcohol as a slightly yellow oil. To a stirred suspension of MnO₂ (13.0 g) in CH₂Cl₂ (20 ml) a solution of the above intermediate in CH₂Cl₂ (5 ml) is added at 4°C. The reaction mixture is stirred for 1 h at 4°C, and filtered over a plug of MgSO₄. The filter is washed several times with CH₂Cl₂ and ethyl acetate, the combined filtrate is evaporated and the residue dried under reduced pressure to yield the title compound (1.51 g, 71.9%) as a slightly brownish solid. ¹H-NMR (300 MHz, CDCl₃): 7.85 (d, J=8.9, 2H); 7.32 (s, 2H); 6.95 (d, J=8.9, 2H); 5.01 (t, J=6.2, 1H); 4.10 (d, J=6.2, 2H); 3.96 (s, MeO); 3.94 (s, 2MeO); 3.80 (s, MeO).

### Example 1b: Propargyl p-methoxyphenylsulfonylamide

To a stirred solution of p-methoxyphenylsulfonyl chloride (8.0 g, 38.71 mmol) and pyridine (7.82 ml, 2.5 equiv.) in CH₂Cl₂ (100 ml) propargylamine (2.73 ml, 1.1 equiv.) is added at 4°C. The reaction mixture is stirred for 6 h at room temperature, and poured onto 1 N aqueous HCl solution and CH₂Cl₂. The organic layer is dried (MgSO₄) and the solvents are evaporated. The residue is suspended in ethyl acetate/hexane (1:8), stirred for 1 h, filtered, and the solid residue washed with ethyl acetate/hexane (1:8) and dried under reduced pressure to yield the title compound (6.1 g, 71 %) as a pale yellowish solid. ¹H-NMR (300 MHz, CDCl₃): 7.86 (d, J=8.9, 2H); 7.00 (d, J=8.9, 2H); 4.65 (br.s, 1 H); 3.89 (s, MeO); 3.84 (~d, J=2.3, 2H); 2.12 (~t, J=2.3, 1H).

### Example 2: 1-p-methoxyphenylsulfonyl-2-(3,4,5-trimethoxyphenyl)-pyrrole

To a solution of 4-bromo-1-p-methoxyphenylsulfonyl-2-(3,4,5-trimethoxyphenyl)-pyrrole (Example 1, 30 mg, 0.062 mmol, 1.0 eq.) in dry THF (1 ml) a solution of 1.7 M tert-butyllithium in hexane (0.05 ml, 0.093 mmol, 1.5 eq.) is added dropwise at -78 °C. The yellow solution is stirred at -78°C for 20 min, then a saturated aqueous NH₄Cl solution (2 ml) is added dropwise at -78°C. The resulting suspension is stirred at 0°C for 10 min, diluted with water (2 ml), and extracted with ethyl acetate (3 x 3 ml). The combined organic layer is dried with Na₂SO₄, filtered and evaporated to afford the crude product as white solid. Pure product (21 mg, 84%) is obtained as a white solid after purification by silica flash chromatography with hexane/ethyl acetate 2:1.
MS: 404.0 (100, [M+H]⁺). HPLC: Rₜ = 3.37 (97%, 254 nm), program 4. ¹H-NMR (300 MHz, DMSO-d₆): 7.47 (m, 1H), 7.32 (d, J = 9.0, 2H), 7.00 (d, J = 9.0, 2H), 6.39 (s, 2H), 6.38 (m, 1H), 6.26 (m, 1H), 3.80 (s, 3H), 3.71 (s, 3H), 3.69 (s, 6H).

### Example 3: N-p-morpholinophenyl-1-p-methoxyphenylsulfonyl-2-(3,4,5-trimethoxyphenyl)-Pyrrole-4-carboxamide

A solution of 1-p-methoxyphenylsulfonyl-2-(3,4,5-trimethoxyphenyl)-pyrrole-4-carboxylic acid (Example 3a, 22.4 mg, 0.05 mmol, 1.0 eq.), p-morpholinoaniline (14 mg, 0.075 mmol, 1.5 eq.) and 4-dimethylaminopyridine (12.2 mg, 0.1 mmol, 2.0 eq.) in dry dichloromethane (1 ml) is cooled to 0°C, and N-(3-dimethylaminopropyl)-N'-ethyl-carbodiimide hydrochloride (12 mg, 0.06 mmol, 1.2 eq.) is added in one portion. The mixture is stirred at room temperature for 48 h. Water (1 ml) is added, the organic layer is separated, the aqueous layer is extracted with dichloromethane (2 x 3 ml), and the combined organic layers are dried with Na₂SO₄, filtered, and evaporated. Pure product (9 mg, 30%) is obtained as a slightly brown solid after purification on silica TLC plate with dichloro-methane/methanol 15:1. MS: 608.0 (100, [M+H]⁺). HPLC: Rₜ = 2.33 (85%, 254 nm) program 3. ¹H-NMR (300 MHz, DMSO-d₆): 9.75 (s, 1H), 8.28(d, J = 2.1, 1H), 7.59 (d, J = 9.0, 2H), 7.37 (d, J = 9.0, 2H), 7.03 (d, J = 9.0, 2H), 6.94 (d, J = 9.1, 2H), 6.69 (d, J = 1.9, 1H), 6.39 (s, 2H), 3.82 (s, 3H), 3.73 (s + t, 7H), 3.69 (s, 6H), 3.07 (t, J = 4.7, 4H).

### Example 3a: 1-p-methoxyphenylsulfonyl-2-(3,4,5-trimethoxyphenyl)-pyrrole-4-carboxylic acid

To a solution of 4-bromo-1-p-methoxyphenylsulfonyl-2-(3,4,5-trimethoxyphenyl)-pyrrole (Example 1, 100 mg, 0.207 mmol, 1.0 eq.) in dry THF (1.5 ml) a solution of 1.7 M tert-butyllithium in hexane (0.18 ml, 0.3 mmol, 1.45 eq.) is added dropwise at -78 °C. The resulting yellow solution is stirred at -78°C for 20 min, and then an excess of dry ice is added in one portion. The mixture is stirred at -78°C for 1 h, and at 0°C for 30 min. 1.0 N aqueous HCI (1 ml) is added, the mixture is extracted with ethyl acetatec (4 x 3 ml), and the combined organic layers are dried (Na₂SO₄), filtered and evaporated to afford the crude product as a foam (93 mg). This product is directly used in next step without purification.

### The following compounds are prepared according to Examples 1-3:

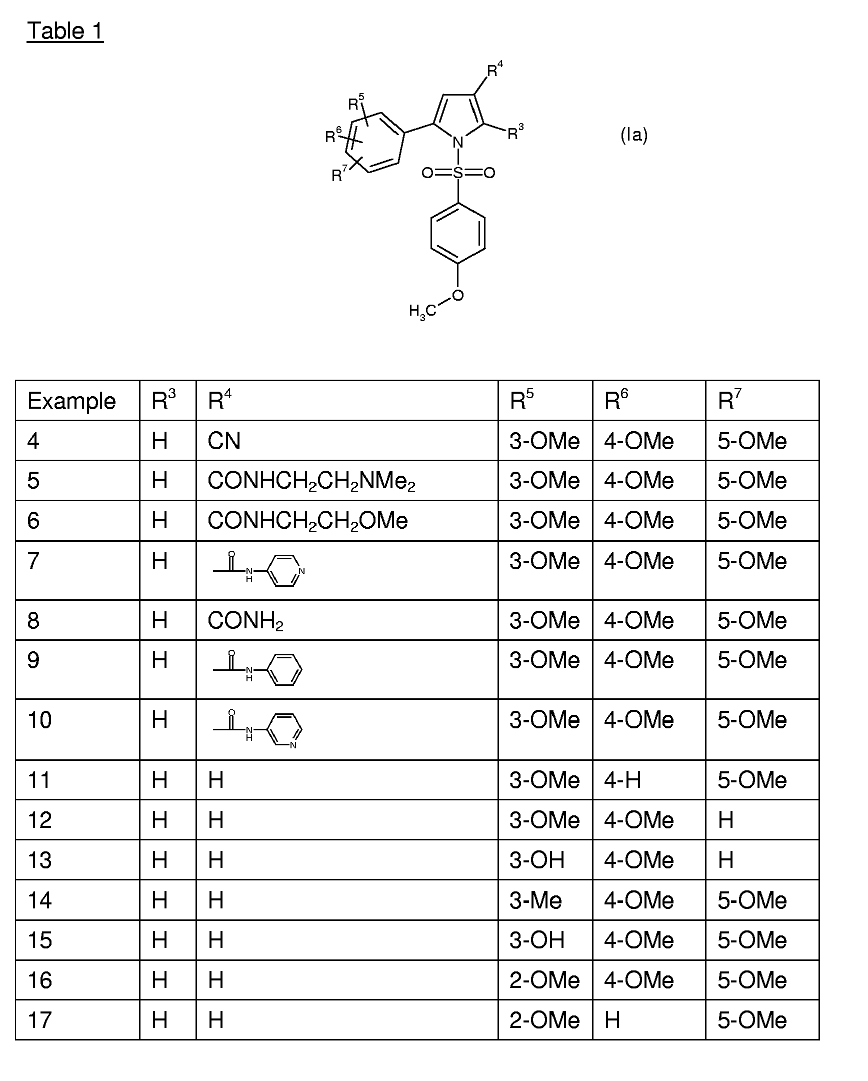

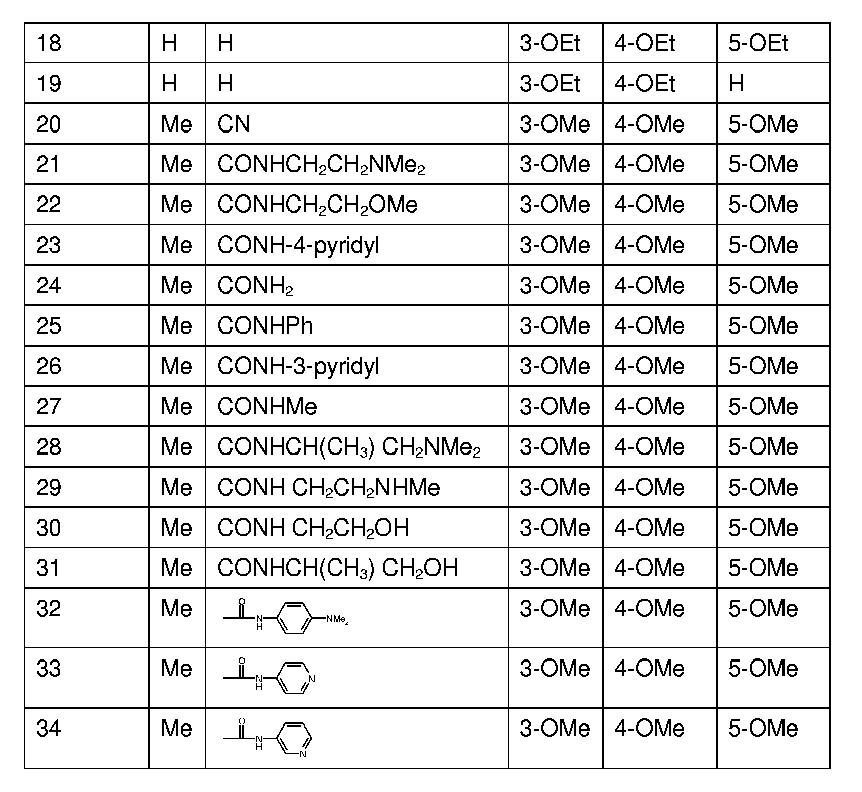

General methods for testing of compounds of the invention:

### Example 35: Cell cultures and cell lines.

Cell lines are cultured in RPMI-1640 tissue culture medium containing either 5% or 10% fetal calf serum, 0.05 mM 2-mercaptoethanol, 2 mM glutamine and penicillin/streptomycin 50 □g/ml (complete medium) (Sigma, Buchs, Switzerland). General growth conditions are 37°C and 7.5% CO₂.
The following mouse cell lines (either EGFP transfected or not) are being used: A20.2J (ATCC: TIB-208), MC57G (ATCC: CRL-2295).
The following human cell lines (either EGFP transfected or not) are being used: HeLa (ATCC: CCL-2), KB (ATCC: CCL-17), MCF7 (ATCC: HTB-22), SK-BR-3 (ATCC: HTB-30), SK-Mel 1 (ATCC: HTB-67), SK-Mel 28 (ATCC: HTB-72), PC-3 (ATCC: CRL-1435), SW 480 (ATCC: CCL-228), NCI-H460 (ATCC: HTB-177), NCI-H1792 (ATCC: CRL-5895), HT1080 (ATCC: CCL-21), Jurkat (ATCC: TIB-152), Ramos (ATCC: CRL-1596), Raji (ATCC: CCL-86), H9 (ATCC: HTB-176), Hut78 (ATCC: TIB-161), K562 (ATCC: CCL 243), HL-60 (ATCC: CCL 240), U-87MG (ATCC: HTB-14), HepG2 (ATCC: HB-8065), U-2 OS (ATCC: HTB-96), Saos-2 (ATCC: HTB-85), U937 (ATCC: CRL 1593), Hs 578T (ATCC: HTB 126), HBL-100 (ATCC: HTB 124), Molt-4 (ATCC: CRL 1582).

### Example 36: Primary screening setup

All the manipulations are performed under sterile conditions. The assays are being performed in commercially available 96 or 384 well flat bottom clear microtiter plates (Greiner, Germany) respectively, which are suitable for tissue culture techniques. A defined number of EGFP transfected adherent test cells (96 well plates: 10⁴ - 10⁵, 384 well plates: 1500 - 2^{*}10⁴) are plated out 24 hours before treatment either in 75 µl (96 well plates) or 60 µl (384 well plates) complete medium per well in order to ensure appropriate cell spreading. For this purpose a peristaltic pump (e.g. Multidrop by Thermo-Labsystems, Finland) or another suitable device is used. Cells in suspension are plated out according to the same procedure but 1 h prior to treatment. Between seeding out and treatment or addition of compounds the cells are incubated at 37°C under 7.5% CO₂. Subsequently, the compounds under investigation are added at defined concentrations (40 - 80 µM in either 25 µl (96 well plates) or 20 µl (384 well plates) complete medium containing max 4% DMSO) with an appropriate device (e.g. liquid handling system, multi channel pipette etc.) resulting in a final concentration in the test well of 10 - 20 µM compound in max 1% DMSO.
Immediately after the addition of the compounds to the cells the zero fluorescence value (t = 0 h) is determined by using a fluorescence microplate reader in order to be able to normalize the fluorescence activities. Afterwards, the test plates are further incubated for a total of 48 h at 37°C under 7.5% CO₂ and are shortly removed only for the purpose of measurement at 8 h, 24 h and 48 h, respectively.

### Example 37: Measurement and quantification of the primary screening.

Relative fluorescence activities of EGFP in compound treated test cells in relation to control cells and cells treated with standard drugs are measured by using a BMG Fluostar microplate fluorescence reader equipped with a filter pair for excitation/emission at 485 nm / 520 nm. The optimum signal to noise ratio is detected by using the time-resolved mode of measurement with a delay of 20 µs and an integration time over 1 ms. The gain is adjusted in such a way that the control cells produce a fluorescence activity of 90% of the maximum. Kinetics is performed by measuring the relative fluorescence activities at t = 0 h, 8 h, 24 h and 48 h. Crude fluorescence activities are individually normalized for different cell numbers and various optical activities of the test compounds / plate-wells by dividing each value from t = 8 h, 24 h and 48 h by the value of t = 0 h resulting in E(8), E(24) and E(48) values. Subsequently, the E(x) values are further processed by forming the inverse (Q-value) of the products E(8)*E(24)*E(48) which result in numbers > 1 for apoptotic / necrotic activities of the compounds and numbers < 1 for proliferative activities of the compounds. Controls (untreated) show values similar to 1. Compounds producing Q values > 2 are being considered relevant in terms of apoptotic / necrotic activity and are subsequently tested in the secondary screening setup.

### Example 38: Secondary screening setup.

All the manipulations are performed under sterile conditions. The assays are being performed in case of adherent cells in commercially available 24 well flat bottom tissue culture plates (Greiner, Germany) and in case of suspension cells in polypropylene tubes (P-tubes) 1.4 ml (Matrix, UK), respectively.
Adherent test cells: 2*10⁴- 4*10⁴ of EGFP transfected cells in 0.5 ml complete medium are plated out 24 h before treatment. At t = 0 the medium is removed and 450 µl new complete medium is added. Subsequently, 50 µl complete medium containing the test compound in max. 5% DMSO is added resulting in final concentrations of 20 µM, 10 µM, 3 µM,1 µM and 0.3 µM of the test compounds, respectively. After 48 h incubation the cells are harvested and analyzed with fluorescence activated cell scanning device (FACSCalibur™, BD Biosciences) according to standard procedures.
Suspension cells: 10⁵ test cells in 450 µl complete medium are pipetted into P-tubes. 50 µl complete medium containing the compounds (see adherent cells) is added immediately. After 48 h of incubation the test cells are analyzed directly on a FACSCalibur™.

### Example 39: Quantification of the secondary screening.

By monitoring the EGFP fluorescence activity in FL1 on a FACSCalibur™, it is possible to distinguish between proliferating cells, apoptotic cells and necrotic cells within the same cell population. The proliferating cells show a high GFP fluorescence activity, the apoptotic population shows an intermediate fluorescence activity whereas the necrotic cells demonstrate a residual fluorescence activity comparable to mock-transfected cells. Within the CeIIQuest Software (BD Biosciences) three regions are defined in the histogram: M1 comprising the proliferating cells, M2 comprising the apoptotic cell population and M3 comprising the necrotic cell population. As readout the relative abundance of the cells belonging either to M1, M2 or M3 are expressed. Compounds inducing M2 values > 50% and M3 values < 30% are being considered relevant and are further tested and characterized in the tertiary / advanced screening setup.

### Example 40: Tertiary screening setup

### A) Hoechst 33342 nuclear staining

This assay is performed in 96 well tissue culture plates. Appropriate number of cells (adherent cells: 3 - 5*10³, suspension cells: 8 - 10*10³) are being seeded out in 80 µl complete medium. Adherent cells are incubated for 24 h for proper spreading out before addition of test compounds while suspension cells are immediately treated with test compounds after seeding out. The test compounds are added in 20 µl complete medium containing max 5% DMSO. The final compound concentrations in the assays are 10 µM, 3 µM,1 µM and 0.3 µM, respectively. After 24 h or 48 h incubation at culture conditions, 10 µl medium containing Hoechst 33342 dye (Sigma B-2261) at 2-5 µg/ml are added to each well. The assay plates are then further incubated for 30 minutes and subsequently analyzed with a standard inverted fluorescence microscope.

The readout allows the determination of the fraction of apoptotic nuclei as well as other morphological criteria specific for apoptosis as a function of the treatment. Results are indicated in Table 2.

**Table 2: Hoechst 33342 nuclear staining (48 hour read-out)**

| Example | Conc | Jurkat | Jily | PBL | HeLa | H460 |
|---|---|---|---|---|---|---|
| 1 | 1µM | 2 | 2 | 0 | 2 | 2 |
| | 0.1µM | 2 | 2 | 0 | 0 | 2 |
| | 0.01µM | 0 | 0 | 0 | 0 | 0 |
| 2 | 1µM | 2 | 2 | 0 | 2 | 2 |
| | 0.1µM | 2 | 2 | 0 | 2 | 2 |
| | 0.01µM | 0 | 0 | 0 | 0 | 0 |
| 3 | 1µM | 2 | 2 | 0 | 2 | 2 |
| | 0.1µM | 2 | 2 | 0 | 2 | 2 |
| | 0.01µM | 2 | 2 | 0 | 0 | 0 |

| | | | | | | |
|---|---|---|---|---|---|---|
| Scoring system: | | | | | | |
| 0: no activity | | | | | | |
| 1: weak activity comprising less than 50% of the cells | | | | | | |
| 2: strong activity comprising more than 50% of the cells. | | | | | | |

### B) MTS proliferation assay

The assay is performed in 96 well tissue culture plates. The cells (range : 1.5*10³ - 10⁴) are seeded out in 80 µl complete medium 24 h prior to compound treatment. The test compounds are added in 20 µl complete medium containing max 5% DMSO. The final compound concentrations in the assays are 10 µM, 3 µM, 1 µM and 0.3 µM, respectively. The assay plates are incubated for 72 h at culture conditions. The MTS reagent is prepared according to the manufacturer's protocol (Promega G1111). 20 µl MTS reagent are added to each well, the assay plates are quickly spun and incubated for another 3 h at culture conditions. Subsequently, the plates are shortly shaked and absorption measured with a microplate-reader at 492 nm. IC₅₀ values are determined by graphical analysis and are indicated in the Table 3.

**Table 3: MTS proliferation assay**

| Example | Jurkat | Jily | HeLa | H460 | MRC5 |
|---|---|---|---|---|---|
| 1 | 2 | 1 | 1 | 1 | 1 |
| 2 | 2 | 2 | 1 | 2 | 2 |
| 3 | 2 | 2 | 2 | 2 | 2 |

| | | | | | |
|---|---|---|---|---|---|
| Scoring system: | | | | | |
| 0: IC₅₀ > 1 µM | | | | | |
| 1: 0.1 µM < IC₅₀ <1 µM | | | | | |
| 2: IC₅₀ < 0.1 µM. | | | | | |

### D) PI staining for cell cycle distribution

1 - 2*10⁵ cells are seeded into 24 well tissue culture plates and incubated for 24 h prior to compound addition. Compounds are added for 24 h in a final concentration of 3 µM or 10 µM. Adherent cells are harvested by trypsinization. The cell suspensions are fixed by adding 2 parts ice cold ethanol 100% while vortexing. Then the samples are stored for > 2 h at -20°C. Subsequently the cells are washed with PBS once and resuspended in 250 µl PBS containing 50 µg/ml PI (Calbiochem # 537059), then the samples are incubated at 37°C for 30 minutes and subsequently analyzed on a FACSCalibur™ monitoring linear PI fluorescence activity on FL2. The readout allows the detection of a possible direct or indirect influence of the tested compounds on the cell cycle. All compounds arrest the cells in the G2M phase.

### Example 41: Soft Capsules

5000 soft gelatin capsules, each comprising as active ingredient 0.05 g of one of the compounds of formula (I) mentioned in the preceding Examples, are prepared as follows: 250 g pulverized active ingredient is suspended in 2 liter Lauroglykol□ (propylene glycol laurate, Gattefossé S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. Compounds of formula (I) 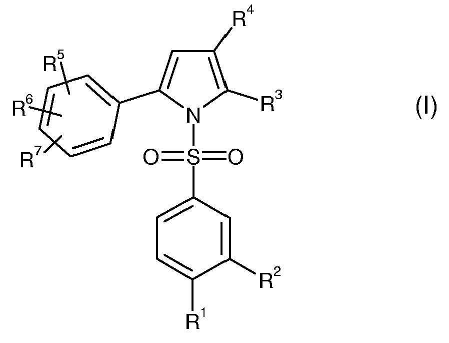 wherein
R¹ represents lower alkyl, halo-lower alkyl, cycloalkyl, hydroxy, lower alkoxy, halo-lower alkoxy, cycloalkoxy or cyano;
R² represents hydrogen, halogen, lower alkyl, hydroxy or lower alkoxy;
or R¹ and R² together with the carbon atoms they are bound to form a 5, 6 or 7 membered carbocyclic or heterocyclic ring;
R³ represents hydrogen, lower alkyl, lower alkoxy-lower alkyl or halo-lower alkyl;
R⁴ represents hydrogen, lower alkyl, a carbamoyl group C(O)NHR⁸, a sulfamoyl group SO₂NHR⁸, halogen or cyano;
R⁵, R⁶ and R⁷, independently of each other, represent hydrogen, lower alkyl, halo-lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, halo-lower alkoxy-lower alkyl, heterocyclyl, heterocyclyl-lower alkyl, optionally substituted phenyl, optionally substituted phenyl-lower alkyl, optionally substituted heteroaryl, optionally substituted heteroaryl-lower alkyl, optionally substituted alkenyl, optionally substituted alkinyl, hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy, sulfamoyloxy, carbamoyloxy, lower alkyl-carbonyloxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl;
lower alkylcarbonyl, cycloalkylcarbonyl, optionally substituted phenylcarbonyl, optionally substituted heteroarylcarbonyl, heterocyclylcarbonyl,
carboxy, lower alkoxycarbonyl, hydroxy-lower alkoxycarbonyl, lower alkoxy-lower alkoxycarbonyl, optionally substituted phenyl-lower alkoxycarbonyl, cyano,
mercapto, lower alkylmercapto, optionally substituted phenylmercapto, lower alkylsulfinyl, halo-lower alkylsulfinyl, optionally substituted phenylsulfinyl, lower alkylsulfonyl, halo-lower alkylsulfonyl, optionally substituted phenylsulfonyl, optionally substituted phenyl-lower alkylsulfonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
for use as medicaments.

2. Compounds of formula (I) according to claim 1, wherein
R¹ represents lower alkyl, cycloalkyl, hydroxy, lower alkoxy, cycloalkoxy or cyano;
R² represents hydrogen;
R³ represents hydrogen, lower alkyl, lower alkoxy-lower alkyl or halo-lower alkyl;
R⁴ represents hydrogen, lower alkyl, a carbamoyl group C(O)NHR⁸, a sulfamoyl group SO₂NHR⁸, halogen or cyano;
R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, heterocyclyl-lower alkyl, optionally substituted phenyl, optionally substituted phenyl-lower alkyl, optionally substituted heteroaryl, optionally substituted heteroaryl-lower alkyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower
alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl;
lower alkylcarbonyl, cycloalkylcarbonyl, optionally substituted phenylcarbonyl, optionally substituted heteroarylcarbonyl, heterocyclylcarbonyl,
carboxy, lower alkoxycarbonyl, hydroxy-lower alkoxycarbonyl, lower alkoxy-lower alkoxycarbonyl, optionally substituted phenyl-lower alkoxycarbonyl, cyano,
mercapto, lower alkylmercapto, optionally substituted phenylmercapto, lower alkylsulfinyl, halo-lower alkylsulfinyl, optionally substituted phenylsulfinyl, lower alkylsulfonyl, halo-lower alkylsulfonyl, optionally substituted phenylsulfonyl, optionally substituted phenyl-lower alkylsulfonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof;
for use as medicaments.

3. Compounds of formula (I) 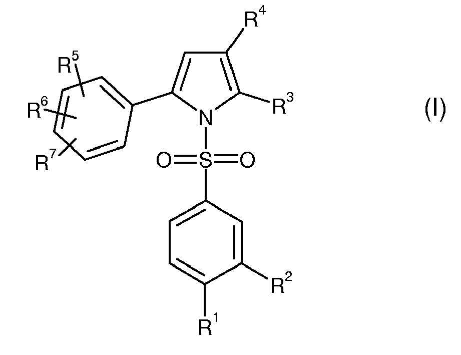 wherein
R¹ represents lower alkyl, cycloalkyl, hydroxy, lower alkoxy, cycloalkoxy or cyano;
R² represents hydrogen;
R³ represents hydrogen, lower alkyl or lower alkoxy-lower alkyl;
R⁴ represents hydrogen, lower alkyl, a carbamoyl group C(O)NHR⁸, a sulfamoyl group SO₂NHR⁸ or cyano;
R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, optionally substituted heteroaryl, optionally substituted heteroaryl-lower alkyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
carbamoyl or sulfamoyl, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heteroaryl or heterocyclyl;
lower alkylcarbonyl, cycloalkylcarbonyl, optionally substituted phenylcarbonyl, optionally substituted heteroarylcarbonyl, heterocyclylcarbonyl, halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof.

4. Compounds of formula (I) according to claim 3, wherein
R¹ represents lower alkyl, hydroxy or lower alkoxy;
R² represents hydrogen;
R³ represents hydrogen, lower alkyl or lower alkoxy-lower alkyl;
R⁴ represents hydrogen, lower alkyl, a carbamoyl group C(O)NHR⁸ or cyano;
R⁵, R⁶ and R⁷, independently of each other, represent hydrogen, lower alkyl, halo-lower alkyl, cycloalkyl, lower alkoxy-lower alkyl, lower alkoxy-lower alkoxy-lower alkyl, heterocyclyl, optionally substituted alkenyl, optionally substituted alkinyl,
hydroxy, lower alkoxy, optionally substituted alkenyloxy, optionally substituted alkinyloxy, halo-lower alkoxy, cycloalkoxy, cycloalkyl-lower alkoxy, hydroxy-lower alkoxy, lower alkoxy-lower alkoxy, heterocyclyloxy, heterocyclyl-lower alkoxy, optionally substituted phenyloxy, optionally substituted phenyl-lower alkoxy, optionally substituted heteroaryloxy, optionally substituted heteroaryl-lower alkoxy,
amino, amino-lower alkyl or amino-lower alkylamino, wherein in each case the nitrogen atom is unsubstituted or substituted by one or two substitutents selected from lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl, optionally substituted alkenyl, lower alkylcarbonyl, lower alkoxycarbonyl and lower aminocarbonyl, or wherein the two substituents on nitrogen form together with the nitrogen heteroaryl or heterocyclyl;
halogen, and cyano;
and wherein two adjacent substituents R⁵ and R⁶ or R⁶ and R⁷ together with the carbon atoms they are bound to may form a 5, 6 or 7 membered carbocyclic or heterocyclic ring; with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl, aminoalkyl, wherein the nitrogen can be substituted by up to two substituents selected from lower alkyl, cycloalkyl, cycloalkyl-lower alkyl, hydroxy-lower alkyl, lower alkoxy-lower alkyl, hydroxy-lower alkoxy-lower alkyl and lower alkoxy-lower alkoxy-lower alkyl; optionally substituted phenyl, optionally substituted phenyl-lower alkyl, optionally substituted heteroaryl, optionally substituted heteroaryl-lower alkyl, or heterocyclyl;
and salts thereof.

5. Compounds of formula (I) according to claim 3, wherein
R¹ represents hydroxy or lower alkoxy;
R² represents hydrogen;
R³ represents hydrogen, lower alkyl or lower alkoxy-lower alkyl;
R⁴ represents hydrogen, lower alkyl, a carbamoyl group C(O)NHR⁸ or cyano;
R⁵, R⁶ and R⁷, independently of each other, represent hydrogen, lower alkyl, lower alkoxy-lower alkyl, hydroxy, lower alkoxy, cycloalkoxy, hydroxy-lower alkoxy, lower alkoxy-lower alkoxy, amino, amino-lower alkyl or amino-lower alkylamino, wherein in each case the nitrogen atom is unsubstituted or substituted by one or two substitutents selected from lower alkyl, hydroxy-lower alkyl, lower alkoxy-lower alkyl, hydroxy-lower alkoxy-lower alkyl, lower alkylcarbonyl, lower alkoxycarbonyl and lower aminocarbonyl, or wherein the two substituents on nitrogen form together with the nitrogen heteroaryl or heterocyclyl; halogen, and cyano;
with the proviso that at least one of R⁵, R⁶ and R⁷ is different from hydrogen;
R⁸ represents hydrogen, alkyl, cycloalkyl, cycloalkylalkyl, hydroxyalkyl, alkoxyalkyl, hydroxyalkoxyalkyl, alkoxyalkoxyalkyl, optionally substituted alkenyl, optionally substituted alkinyl; optionally substituted phenyl, optionally substituted phenyl-lower alkyl, optionally substituted heteroaryl, optionally substituted heteroaryl-lower alkyl, or heterocyclyl;
and salts thereof.

6. Compounds of formula (I) according to claim 3, wherein
R¹ is methoxy;
R² is hydrogen;
R³ is hydrogen;
R⁴ is hydrogen or a carbamoyl group C(O)NHR⁸;
R⁵, R⁶ and R⁷ represent methoxy and are located in position 3, 4 and 5 of the phenyl ring; and R⁸ is p-morpholinophenyl.

7. A method of manufacture of a compound of formula (1) according to claim 1 wherein
A) a pyrrole of formula (II) 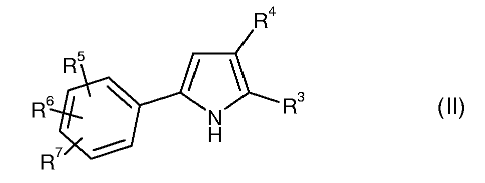 is reacted with a sulfonyl chloride of formula (III) 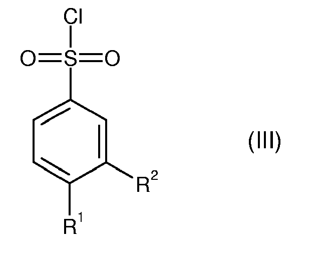 wherein the substituents are as defined for a compound of formula (I), or a derivative thereof wherein functional groups are in protected form, in the presence of a strong base and an inert solvent, and any protecting groups in a protected derivative of a compound of the formula (I) are removed;
B) a sulfonamide of formula (IV) 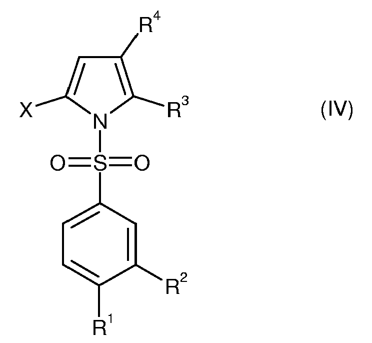 is reacted with a compound of formula (V) 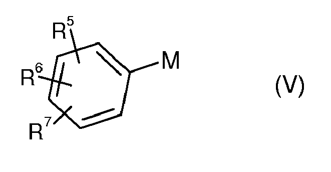 wherein the substituents R¹ to R⁷ are as defined for a compound of formula (I), X represents halogen and M represents alkali metal, substituted silicon or boronic acid, or a derivative thereof wherein functional groups are in protected form, and any protecting groups in a protected derivative of a compound of the formula (I) are removed;
C) a sulfonamide of formula (VI) 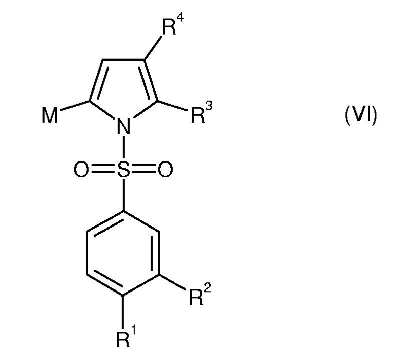 is reacted with a compound of formula (VII) 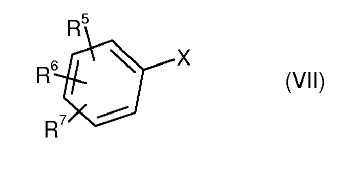 wherein the substituents R¹ to R⁷ are as defined for a compound of formula (I), X represents halogen and M represents alkali metal, substituted silicon or boronic acid, or a derivative thereof wherein functional groups are in protected form, and any protecting groups in a protected derivative of a compound of the formula (I) are removed; or
D) for the preparation of a compound of formula (I) wherein R³ is hydrogen and R⁴ is chlorine or bromine, a benzoyl acetylene of formula (VIII) 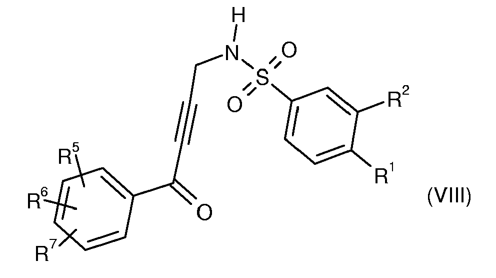 wherein R¹, R², R⁵, R⁶ and R⁷ are as defined for a compound of formula (I), or a derivative thereof wherein functional groups are in protected form, is reacted with hydrochloric or hydrobromic; or
E) for the preparation of a compound of formula (I) wherein R⁴ represents a carbamoyl group C(O)NHR⁸ or a sulfamoyl group SO₂NHR⁸, a compound of formula (I) wherein R⁴ is a carboxylic acid or sulfonic acid residue or the corresponding acid halide, or a derivative thereof wherein functional groups are in protected form, is reacted with an amine of formula (IX)
R⁸NH₂ (IX)
wherein R⁸ has the meaning as defined under formula (I), or a derivative thereof wherein functional groups are in protected form;
and, if so desired, an obtainable compound of formula (I) is converted into another compound of formula (I), a free compound of formula (I) is converted into a salt, an obtainable salt of a compound of formula (I) is converted into the free compound or another salt, and/or a mixture of isomeric compounds of formula (I) is separated into the individual isomers.

8. A pharmaceutical composition comprising a compound of formula (I) according to claim 1 and a pharmaceutically acceptable carrier.

9. Use of a compound of formula (I) according to claim 1 or of a prodrug or a pharmaceutically acceptable salt of such a compound for the preparation of a pharmaceutical composition for the treatment of a neoplastic disease, autoimmune disease, transplantation related pathology and/or degenerative disease.

10. A method for the treatment of a neoplastic disease, autoimmune disease, transplantation related pathology and/or degenerative disease, which comprises administering a compound of formula (I) as defined in claim 1 or of a prodrug or a pharmaceutically acceptable salt thereof, in a quantity effective against said disease, to a warm-blooded animal requiring such treatment.
